# EUROPÄISCHE PATENTSCHRIFT

(11) **EP 4 473 907 B1**
(45) Veröffentlichungstag und Bekanntmachung des Hinweises auf die Patenterteilung: **08.07.2026**
(21) Anmeldenummer: 24174109.9
(22) Anmeldetag: 03.05.2024
(51) Int. Cl.: A61B 5/103, A61B 5/11

(54) **VERFAHREN ZUR ERFASSUNG EINES PERFORMANCEDATENSATZES FÜR EINE BEWEGUNG**
METHOD FOR DETECTING A PERFORMANCE DATASET FOR A MOVEMENT
PROCÉDÉ DE DÉTECTION D'UN ENSEMBLE DE DONNÉES DE PERFORMANCE POUR UN MOUVEMENT

(30) Priorität: 08.06.2023 DE 102023205355
(43) Veröffentlichungstag der Anmeldung: 11.12.2024
(73) Patentinhaber: ContiTech Deutschland GmbH, 30175 Hannover (DE)
(72) Erfinder: Werhahn, Max Konstantin, 30175 Hannover (DE); Marienfeld, Peter Michael, 30175 Hannover (DE); Plinke, Moritz, 30175 Hannover (DE); Teichrib, Alexander, 30175 Hannover (DE)
(74) Vertreter: Preusser, Andrea

(56) Entgegenhaltungen:
- US-A1- 2014 019 063
- US-A1- 2019 298 226
- LI BOCHEN ET AL: "Analysis of Plantar Pressure Image Based on Flexible Force-Sensitive Sensor Array", 2020 13TH INTERNATIONAL SYMPOSIUM ON COMPUTATIONAL INTELLIGENCE AND DESIGN (ISCID), IEEE, 12 December 2020 (2020-12-12), pages 326 - 329, XP033881005, DOI: 10.1109/ISCID51228.2020.00079

## Beschreibung

Die vorliegende Erfindung betrifft ein computerimplementiertes Verfahren zur Erfassung eines Performancedatensatzes für eine von einem Lebewesens, insbesondere einem Huftier oder einem Menschen, durchgeführte Bewegung. Genauer betrifft die Erfindung ein Verfahren zur Erfassung eines solchen Performancedatensatzes auf Basis eines mittels einer auf der Unterseite zumindest eines Fußes des Lebewesens angeordneten Sensoreinrichtung gemessenen Reaktionskraftdatensatzes.

Auf diversen Gebieten des Sports oder der Medizin hat es sich als vorteilhaft herausgestellt, die Performance von Menschen oder Tieren bei einer Bewegung durch Aufzeichnen und Auswerten der beim Auftreffen des Fußes des Menschen oder des Tieres auf den Boden wirkenden Kräfte zu analysieren. Zur Erfassung der Bodenreaktionskraftverteilung über die Bodenkontaktfläche des Fußes Menschen oder des Fußes eines Huftiers sind unterschiedliche Reaktionskraft-Sensoreinrichtungen bekannt. Derartige Sensoreinrichtungen können beispielsweise als Einlegesohle ausgeführt oder in einen Schuh, Hufschuh oder in ein Hufeisen integriert sein.

Aus der GB 2 482 192 B ist es bekannt, Kraftsensoren für derartige Zwecke an einem Hufeisen zu befestigen. Die DE 102011 016 344 A1 zeigt Kraftsensoren, die in einer Einlegesohle für einen Hufschuh eingebettet sind. Li Bochen et al., "Analysis of Plantar Pressure Image Based on Flexible Force-Sensitive Sensor Array", 2020, beschreibt ein Verfahren zur Analyse von Fußsohlendruckbildern mittels eines flexiblen Kraftsensorarrays auf einer stationären Plattform, das die Extraktion von Gangmerkmalen für klinische Diagnosen ermöglicht. US 2014/0019063 A1 offenbart ein Sensorsystem mit Drucksensoren in einem Hufeisen zur Messung physiologischer Eigenschaften wie Druck am Huf eines Tieres, wobei die Daten drahtlos übertragen und in Echtzeit analysiert werden. US 2019/0298226 A1 beschreibt ein System zur Lahmheitserkennung bei Vierbeinern unter Verwendung von Sensoren in Fußbekleidung, die Kraftdaten pro Gliedmaße erfassen und auf Asymmetrien zwischen den Gliedmaßen prüfen.

Informationen über die Verteilung der Reaktionskräfte über die Bodenkontaktfläche und über deren zeitliche Veränderung während einer Bewegung sind zum einen hilfreich, um die Belastung eines Fußes zu bestimmen. Zum anderen können sie auch als diagnostisches Instrument verwendet werden, um die Performance bei einer Sportbewegung zu tracken und zu optimieren oder um Fehlstellungen des Fußes, Lahmheit und/oder andere Krankheitsbilder - möglichst frühzeitig - zu erkennen. Auch ist es anhand dieser Informationen möglich, einen nicht ordnungsgemäßen Sitz des Schuhs, des Hufschuhs oder des Hufeisens zu erkennen. All dies bedarf einer geeigneten Auswertung der aufgezeichneten Reaktionskraftdaten.

Aufgabe der vorliegenden Erfindung ist es, ein Verfahren der eingangs genannten Art bereitzustellen, mit dem eine Auswertung der Reaktionskraftdaten in besonders effizienter und einfach zu implementierender Weise realisiert werden kann. Insbesondere sollen zur Ermittlung präziser Informationen über die Performance einer Bewegung auch Datensätze herangezogen werden können, die anhand einer geringen Anzahl an Sensoren generiert wurden.

Diese Aufgabe wird durch ein Verfahren mit den Merkmalen des Anspruchs 1 gelöst. Bevorzugte Merkmale sind Gegenstand der abhängigen Ansprüche. Weitere Vorteile und Merkmale sind der allgemeinen Beschreibung sowie den Ausführungsbeispielen zu entnehmen.

Das erfindungsgemäße computerimplementierte Verfahren zur Erfassung eines Performancedatensatzes für eine von einem Lebewesen, insbesondere einem Huftier oder einem Menschen, durchgeführte Bewegung enthält folgende Schritte:
- Empfangen eines mittels einer auf der Unterseite zumindest eines Fußes des Lebewesens angeordneten Sensoreinrichtung gemessenen Reaktionskraftdatensatzes,
   wobei die Sensoreinrichtung ein erstes und ein zweites Paar von bezüglich einer Symmetrieebene der Sensoreinrichtung zueinander spiegelsymmetrisch angeordneter Sensoren aufweist,
   wobei der Reaktionskraftdatensatz pro Sensor jeweils einen dem jeweiligen Sensor zugeordneten Sensordatensatz enthaltend einen während der Bewegung aufgezeichneten zeitlichen Verlauf einer mit dem Sensor gemessenen Reaktionskraft aufweist,
- Strukturieren des Reaktionskraftdatensatzes in einer Datenstruktur mit zumindest zwei Dimensionen, wobei entlang einer ersten Dimension die Messzeitpunkte der Reaktionskraft geordnet werden und entlang einer zweiten Dimension die unterschiedlichen Sensordatensätze geordnet werden, derart, dass
   die dem ersten Paar von Sensoren zugeordneten Sensordatensätze bezüglich einer gedachten, in Richtung der ersten Dimension verlaufenden Symmetrieachse spiegelsymmetrisch gegenüberliegend angeordnet sind und
   die dem zweiten Paar von Sensoren zugeordneten Sensordatensätze bezüglich der Symmetrieachse spiegelsymmetrisch gegenüberliegend sowie die dem ersten Paar von Sensoren zugeordneten Sensordatensätze umgebend angeordnet sind;
- Ermitteln von Performancedaten auf Basis der Datenstruktur und speichern in einem Performancedatensatz.

Der Erfindung liegt die Idee zugrunde, eine in der Anordnung der Sensoren der Sensoreinrichtung vorhandene Symmetrie in die Struktur einer die Reaktionskraftdaten enthaltenden Datenstruktur einfließen zu lassen. Die Datenstruktur weist dabei eine gedachte Symmetrieachse auf, die eine Zeitrichtung definiert und um die herum die zeitlich synchronisierten Sensordatensätze angeordnet sind. Insbesondere teilt die Symmetrieachse die Datenstruktur in zwei Hälften. Zwei Sensordatensätze sind dabei genau dann bezüglich der Symmetrieachse der Datenstruktur symmetrisch angeordnet, wenn auch die Sensoren, mit denen die Sensordatensätze aufgezeichnet wurden, symmetrisch bezüglich der Symmetrieebene der Sensoreinrichtung angeordnet sind. Auf diese Weise spiegelt die symmetrische Struktur der Datenstruktur die Symmetrie der Sensoreinrichtung wider. Symmetrien oder Asymmetrien der mit der Sensoreinrichtung gemessenen Reaktionskraftverteilung korrelieren daher stark mit einer Symmetrie oder Asymmetrie der Datenstruktur. Beispielsweise führt eine symmetrische Verteilung der Reaktionskraft zu einer symmetrischen Verteilung der Messwerte in der Datenstruktur.

Symmetrien oder Asymmetrien der Reaktionskraftverteilung können durch Auswertung der geordneten Datenstruktur anhand von computergestützten Methoden deutlicher erkannt werden, wodurch aussagekräftige Auswertungsergebnisse zur Beurteilung der Performance des Lebewesens produzierbar sind. Es hat sich ferner überraschenderweise herausgestellt, dass durch die Verwendung der vorbeschriebenen Datenstruktur eine aussagekräftige Analyse der Performance bereits durch die Verwendung einer geringen Anzahl an Sensordatensätzen, die mittels zueinander spiegelsymmetrisch angeordneten Sensoren aufgezeichnet wurden, möglich ist. Folglich ermöglicht das erfindungsgemäße Verfahren, kompaktere Reaktionskraft-Sensoreinrichtungen für eine Analyse der Performance zu verwenden.

Vorzugsweise sind die dem ersten Paar von Sensoren zugeordneten Sensordatensätze und die dem zweiten Paar von Sensoren zugeordneten Sensordatensätze zeitlich synchronisiert in der Datenstruktur angeordnet.

Das Ermitteln von Performancedaten auf Basis der Datenstruktur umfasst vorzugsweise das Auswerten der Datenstruktur, bevorzugt durch Vergleichen der Datenstruktur mit Referenzen in einer Datenbank und/oder durch Klassifizieren der Datenstruktur und/oder eines oder mehrerer ihrer Merkmale mittels statistischer und/oder analytischer Methoden, wie z.B. einer Varianzanalyse und/oder einer (Support Vector-) Regression. Alternativ oder zusätzlich kann die Auswertung ein Klassifizieren der der Datenstruktur und/oder eines oder mehrerer ihrer Merkmale anhand eines Machine-Learning-Algorithmus (z.B. eines neuronalen Netzwerkes), insbesondere anhand eines vorab mit einer Vielzahl von Referenzen und/oder mittels Deep Learning trainierten Machine-Learning-Algorithmus umfassen.

Die ermittelten Performancedaten können Informationen darüber enthalten, ob und/oder inwieweit das Lebewesen die Bewegung korrekt ausgeführt hat. Die Bewegung kann beispielsweise eine Sportbewegung wie ein Sprung, ein Lauf, ein Wurf, eine Tanzbewegung, eine Stoß-, Schlag- oder Tritttechnik, ein Hieb oder ein Stich mit einer Waffe oder das Spielen eines Balles mit einem Schläger sein. Die ermittelten Performancedaten können alternativ oder zusätzlich Informationen darüber enthalten, ob beim Lebewesen Fehlstellungen des zumindest einen Fußes, Lahmheit und/oder andere Krankheitsbilder oder ein ordnungsgemäßer Sitz des Schuhs, des Hufschuhs oder des Hufeisens vorliegt. Die ermittelten Performancedaten können ferner Informationen über die Bewegungsrichtung bzw. Bewegungspfad der von dem Lebewesen durchgeführten Bewegung enthalten. Beispielsweise kann anhand der Performancedaten ermittelt werden, ob das Lebewesen sich geradeaus, entlang einer Linkskurve oder entlang einer Rechtskurve bewegt.

In einer bevorzugten Ausgestaltung des erfindungsgemäßen Verfahrens ist die Datenstruktur eine Matrix mit zumindest zwei Indizes, wobei Einträge mit dem gleichen ersten Index der Matrix demselben Messzeitpunkt der Reaktionskraft zugeordnet sind und Einträge mit dem gleichen zweiten Index zum gleichen Sensordatensatz gehören. Beispielsweise können die dem ersten bzw. dem zweiten Paar von spiegelsymmetrisch angeordneten Sensoren zugeordneten Sensordatensätze als Spaltenvektor vorliegen. Die dem ersten Paar von Sensoren zugeordneten Sensordatensätze können dann um die in Spaltenrichtung verlaufende Symmetrieachse herum spiegelsymmetrisch gegenüberliegend angeordnet sein. Die dem zweiten Paar von Sensoren zugeordneten Sensordatensätze sind dann ebenfalls um die Symmetrieachse herum spiegelsymmetrisch gegenüberliegend angeordnet, wobei die dem ersten Paar von Sensoren zugeordneten Sensordatensätze zwischen den dem zweiten Paar von Sensoren zugeordneten Sensordatensätzen angeordnet sind.

In einer weiteren bevorzugten Ausgestaltung des erfindungsgemäßen Verfahrens wird auf Basis der Datenstruktur eine graphische Darstellung des Reaktionskraftdatensatzes erzeugt, wobei das Ermitteln von Performancedaten auf Basis der Datenstruktur eine Auswertung der graphischen Darstellung, insbesondere mittels eines Bildauswertealgorithmus, umfasst. Es hat sich herausgestellt, dass die vorbeschriebene Datenstruktur durch eine graphische Auswertung hervorragende Ergebnisse liefert.

In einer weiteren bevorzugten Ausgestaltung des erfindungsgemäßen Verfahrens enthalten die Sensordatensätze jeweils einen über mehrere von dem Lebewesen durchgeführte Bewegungszyklen gemittelten zeitlichen Verlauf der mit dem jeweiligen Sensor aufgezeichneten Reaktionskraft. Auf diese Weise kann eine zyklische Bewegung, wie z.B. ein Lauf, über einen längeren Zeitraum mittels der Sensoreinrichtung vermessen und die gemittelten Messwerte in einer einzigen Datenstruktur zusammengefasst und mit geringem Rechenaufwand ausgewertet werden. Dadurch lassen sich in effizienter Weise präzise Auswerteergebnisse erzeugen.

In einer weiteren bevorzugten Ausgestaltung des erfindungsgemäßen Verfahrens sind das erste Paar und das zweite Paar von Sensoren jeweils auf einem gemeinsamen, während der Messung des Reaktionskraftdatensatzes auf der Unterseite eines Fußes des Lebewesens angeordneten Sensorträger Sensoreinrichtung, angeordnet oder jeweils auf zwei separaten, während der Messung des Reaktionskraftdatensatzes auf der Unterseite unterschiedlicher Füße des Lebewesens angeordneten Sensorträgern der Sensoreinrichtung angeordnet. Als Sensorträger kommen insbesondere Schuhe, Hufeisen oder Hufschuhe oder Einlegesohlen für diese in Betracht. Beispielsweise können das erste bzw. das zweite Paar auf einem Hufeisen angeordnet sein. Das Hufeisen definiert eine Symmetrieebene bezüglich derer die Paare von Sensoren spiegelsymmetrisch angeordnet sind. Alternativ können die beiden Sensoren des ersten bzw. des zweiten Paares jeweils auf einem Schuh eines Schuhpaares angeordnet sein. In diesem Fall wird die Symmetrieebene durch die spiegelsymmetrisch ausgebildeten Schuhe des Schuhpaares definiert.

In einer weiteren bevorzugten Ausgestaltung des erfindungsgemäßen Verfahrens enthält das Verfahren den folgenden ersten Schritt:
- Aufzeichnen des Reaktionskraftdatensatzes mittels einer auf der Unterseite zumindest eines Fußes des Lebewesens angeordneten Sensoreinrichtung.

Insgesamt wird mit dem erfindungsgemäßen Verfahren eine in einfacher Weise implementierbare und automatisierbare sowie zuverlässige Lösung für die Bewertung einer Performance einer Bewegung auf Basis von Reaktionskraftdaten bereitgestellt.

Gegenstand der vorliegenden Erfindung ist ferner ein Computerprogrammprodukt, umfassend Befehle, die bei der Ausführung des Programms durch einen Computer diesen veranlassen, das erfindungsgemäße Verfahren auszuführen.

Gegenstand der vorliegenden Erfindung ist ferner ein Computer, umfassend Mittel zur Ausführung des erfindungsgemäßen Verfahrens.

Es wird ausdrücklich darauf hingewiesen, dass die vorstehend erläuterten Ausgestaltungen der Erfindung jeweils für sich oder in einer beliebigen technisch sinnvollen Kombination auch untereinander jeweils mit dem Gegenstand der unabhängigen Ansprüche kombinierbar sind.

Abwandlungen und Ausgestaltungen der Erfindung sowie weitere Vorteile und Einzelheiten der Erfindung lassen sich der nachfolgenden gegenständlichen Beschreibung und den Zeichnungen entnehmen. In den schematischen Figuren zeigen:
- Fig. 1a: ein erstes Beispiel einer Sensoreinrichtung, mit der ein Reaktionskraftdatensatz gemessen wurde;
- Fig. 1b: ein Beispiel einer Strukturierung des mit der Sensoreinrichtung aus Fig. 1a gemessenen Reaktionskraftdatensatzes in einer Datenstruktur;
- Fig. 2a: ein zweites Beispiel einer Sensoreinrichtung, mit der ein Reaktionskraftdatensatz gemessen wurde;
- Fig. 2b: ein Beispiel einer Strukturierung des mit der Sensoreinrichtung aus Fig. 2a gemessenen Reaktionskraftdatensatzes in einer Datenstruktur;
- Fig. 3: ein weiteres Beispiel einer Datenstruktur;
- Fig. 4: eine graphische Darstellung einer Datenstruktur;
- Fig. 5: ein Ablaufschema einer Ausführungsform des erfindungsgemäßen Verfahrens.

Gleich oder ähnlich wirkende Teile sind - sofern dienlich - mit identischen Bezugsziffern versehen.

Einzelne technische Merkmale der nachbeschriebenen Ausführungsbeispiele können auch in Kombination mit vorbeschriebenen Ausführungsbeispielen sowie den Merkmalen der unabhängigen Ansprüche und etwaiger weiterer Ansprüche zu erfindungsgemäßen Gegenständen kombiniert werden.

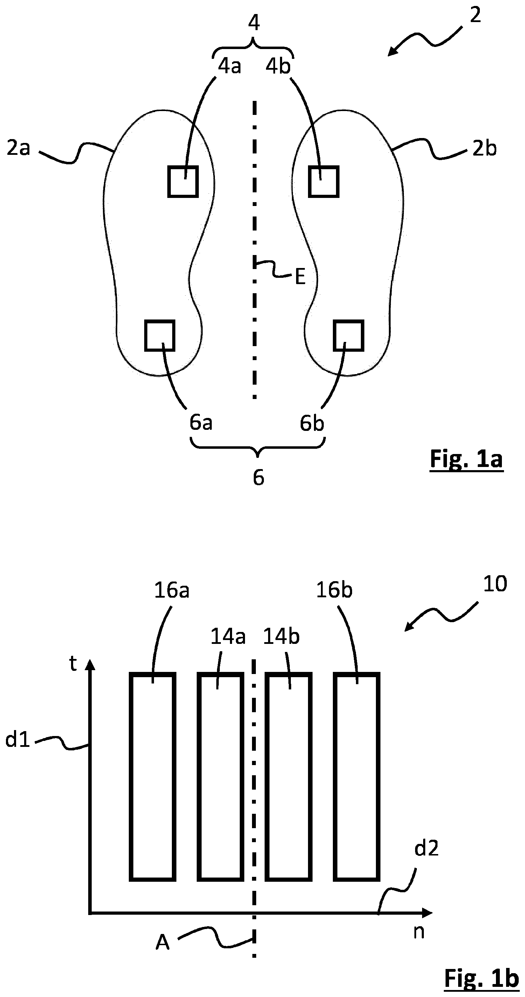

Fig. 1a zeigt ein erstes Beispiel einer Sensoreinrichtung 2, mit der ein Reaktionskraftdatensatz aufgenommen wurde. Die Sensoreinrichtung 2 weist zwei separate Sensorträger 2a, 2b in Form von spiegelsymmetrisch ausgebildeten Schuheinlegesohlen auf, die zur Messung des Reaktionskraftdatensatzes jeweils auf der Unterseite eines Fußes eines Menschen angeordnet werden können. Die spiegelsymmetrisch ausgebildeten Sensorträger 2a, 2b definieren eine Symmetrieebene E, bezüglich der ein erstes Paar 4 von Sensoren 4a, 4b und ein zweites Paar 6 von Sensoren 4a, 4b; 6a, 6b jeweils zueinander spiegelsymmetrisch auf der Sensoreinrichtung 2 angeordnet ist.

Fig. 1b zeigt ein Beispiel einer im Rahmen eines erfindungsgemäßen Ausführungsbeispiels des erfindungsgemäßen Verfahrens durchgeführten Strukturierung des mit der Sensoreinrichtung 2 aus Fig. 1a gemessenen Reaktionskraftdatensatzes in einer Datenstruktur 10 mit zwei Dimensionen d1, d2. Der Reaktionskraftdatensatz weist pro Sensor 4a, 4b; 6a, 6b jeweils einen dem jeweiligen Sensor 4a, 4b; 6a, 6b zugeordneten Sensordatensatz 14a, 14b; 16a, 16b auf. Hierbei ist dem Sensor 4a der Sensordatensatz 14a, dem Sensor 4b der Sensordatensatz 14b, dem Sensor 6a der Sensordatensatz 16a und dem Sensor 6b der Sensordatensatz 16b zugeordnet. Jeder Sensordatensatz 14a, 14b 16a, 16b enthält einen während der Bewegung aufgezeichneten zeitlichen Verlauf einer mit dem jeweiligen Sensor 4a, 4b; 6a, 6b gemessenen Reaktionskraft. Die Sensordatensätze 14a, 14b; 16a, 16b können somit als separate Messkanäle n betrachtet werden.

Der Reaktionskraftdatensatz ist in der Datenstruktur 10 derart strukturiert, dass die Messzeitpunkte t der Reaktionskraft entlang einer ersten Dimension d1 und die unterschiedlichen Sensordatensätze 14a, 14b; 16a, 16b bzw. Messkanäle n entlang einer zweiten Dimension geordnet werden. Hierbei wird die erfindungsgemäße Idee realisiert, eine in der Anordnung der Sensoren 4a, 4b; 6a, 6b vorhandene Symmetrie in die Struktur der Datenstruktur 10 einfließen zu lassen. Dazu werden die dem ersten Paar 4 von Sensoren 4a, 4b zugeordneten Sensordatensätze 14a, 14b bezüglich einer gedachten, in Richtung der ersten Dimension d1 verlaufenden Symmetrieachse A spiegelsymmetrisch gegenüberliegend angeordnet. Des Weiteren werden die dem zweiten Paar 6 von Sensoren 6a, 6b zugeordneten Sensordatensätze 16a, 16b bezüglich der Symmetrieachse A spiegelsymmetrisch gegenüberliegend und derart angeordnet, dass sie die dem ersten Paar 4 von Sensoren 4a, 4b zugeordneten Sensordatensätze umgeben. Auf diese Weise spiegelt die Struktur der Datenstruktur 10 die Symmetrie der Sensoreinrichtung 2 wider. Symmetrien oder Asymmetrien der mit der Sensoreinrichtung 2 gemessenen Reaktionskraftverteilung korrelieren daher stark mit einer Symmetrie oder Asymmetrie der Datenstruktur 10. Beispielsweise führt eine symmetrische Verteilung der Reaktionskraft zu einer symmetrischen Verteilung der Messwerte in der Datenstruktur 10.

In der vorliegenden Ausführungsform teilt die Symmetrieachse A die Datenstruktur 10 in zwei Hälften. Ferner sind die Sensordatensätze 14a, 14b; 16a, 16b in der vorliegenden Ausführungsform zeitlich synchronisiert, d.h. sie beginnen und enden in Richtung der ersten Dimension d1 auf gleicher Höhe. Weist die Sensoreinrichtung 2 weitere Paare von spiegelsymmetrisch angeordneten Sensoren auf, so können die diesen Paaren zugeordneten Sensordatensätze nach dem vorbeschriebenen Prinzip der Datenstruktur hinzugefügt, z.B. paarweise von außen an die vorhandenen Sensordatensätze angelegt werden.

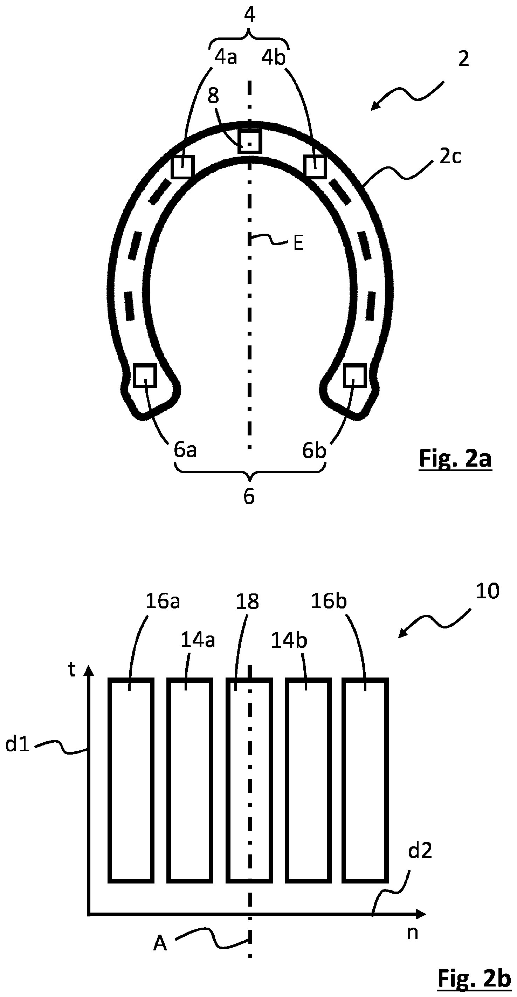

Fig. 2a zeigt ein zweites Beispiel einer Sensoreinrichtung 2, mit der ein Reaktionskraftdatensatz aufgenommen wurde. Die Sensoreinrichtung 2 weist einen Sensorträger 2c in Form eines spiegelsymmetrisch ausgebildeten Hufeisens auf, das zur Messung des Reaktionskraftdatensatzes auf der Unterseite eines Fußes eines Menschen angeordnet werden kann. Der spiegelsymmetrisch ausgebildeten Sensorträger 2c definiert eine Symmetrieebene E, bezüglich der ein erstes Paar 4 von Sensoren 4a, 4b und ein zweites Paar 6 von Sensoren 6a, 6b jeweils zueinander spiegelsymmetrisch auf der Sensoreinrichtung 2 angeordnet ist. Darüber hinaus weist Die Sensoreinrichtung 2 noch einen einzelnen Sensor 8 auf, der zentral auf der Symmetrieebene E angeordnet ist.

Fig. 2b zeigt ein Beispiel einer im Rahmen eines weiteren erfindungsgemäßen Ausführungsbeispiels des erfindungsgemäßen Verfahrens durchgeführten Strukturierung des mit der Sensoreinrichtung 2 aus Fig. 2a gemessenen Reaktionskraftdatensatzes in einer Datenstruktur 10 nach demselben Prinzip wie die in Fig. 1b beschriebene Strukturierung. Im Unterschied zur Datenstruktur 10 der Fig. 1b weist die hier gezeigte Datenstruktur 10 einen dem einzelnen Sensor 8 zugeordneten Sensordatensatz 18 auf, der zentral auf der Symmetrieachse A angeordnet ist. Somit spiegelt auch im vorliegenden Fall die Struktur der Datenstruktur 10 die Symmetrie der Sensoreinrichtung 2 wider.

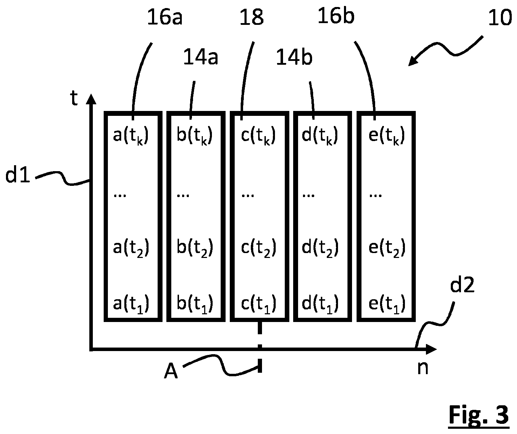

Fig. 3 zeigt ein Beispiel einer als Matrix ausgebildeten Datenstruktur 10, wie sie in sämtlichen erfindungsgemäßen Ausführungsbeispielen verwendet werden kann. Die Matrix hat zumindest zwei Indizes, wobei der erste Index entlang der ersten Dimension d1 gezählt wird und der zweite Indes entlang der zweiten Dimension d2 gezählt wird. Einträge mit dem gleichen ersten Index sind demselben Messzeitpunkt t der Reaktionskraft zugeordnet. Einträge mit dem gleichen zweiten Index gehören zum gleichen Sensordatensatz 14a, 14b; 16a, 16b; 18. Die Sensordatensätze 14a, 14b; 16a, 16b; 18 können somit als Spaltenvektoren a(t), b(t), c(t), d(t), e(t) betrachtet werden. Selbstverständlich ist auch eine Anordnung als Zeilenvektor entlang einer horizontal verlaufenden Symmetrieachse A denkbar.

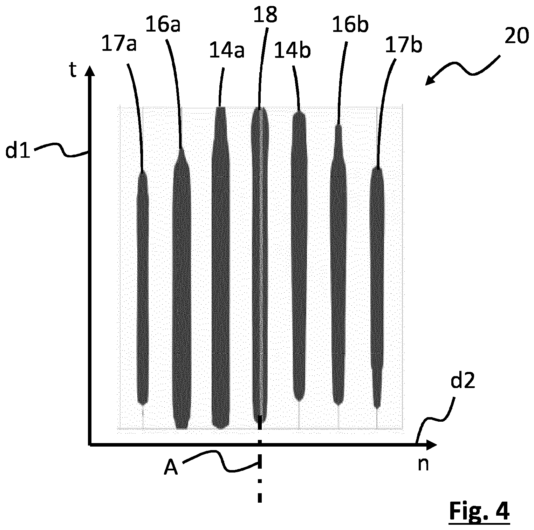

Fig. 4 zeigt ein Beispiel einer graphischen Darstellung 20 des Reaktionskraftdatensatzes, wie sie in sämtlichen erfindungsgemäßen Ausführungsbeispielen verwendet werden kann. Der Reaktionskraftdatensatz wurde mit einem als Hufeisen ausgebildeten Sensorträger 2c aufgezeichnet, der im Wesentlichen so aufgebaut ist, wie der in Fig. 2a beschriebene Sensorträger 2c, wobei der Sensorträger ferner ein drittes Paar von spiegelsymmetrisch angeordneten Sensoren aufweist. Die Sensordatensätze 17a, 17b sind diesem dritten Paar von Sensoren zugeordnet und als Paar von außen an die Sensordatensätze 14a, 14b; 16a, 16b; 18 hinzugefügt bzw. angelegt. Die Sensordatensätze 14a, 14b; 16a, 16b; 17a, 17b; 18 sind in einer Weise visualisiert, dass die Messwerte der Reaktionskraft als Strichbreite dargestellt sind. Andere Visualisierungen, z.B. ein 2D-Farbplot, sind ebenfalls denkbar. Die hier betrachtete Bewegung ist ein Lauf eines Pferdes, zu dem über ca. 60 Sekunden lang die Reaktionskraft zwischen dem Fuß bzw. Huf des Pferdes und dem Boden gemessen wurde. Der Lauf ist eine zyklische Bewegung bestehend aus mehreren Schritten (Bewegungszyklen). Die Sensordatensätze 14a, 14b; 16a, 16b; 17a, 17b; 18 wurden über sämtliche Schritte gemittelt, bevor sie in der Datenstruktur 10 strukturiert wurden, auf deren Basis die graphische Darstellung 20 erstellt wurde. Es lässt sich auf den ersten Blick erkennen, dass die Reaktionskraftverteilung asymmetrisch ist. Eine solche grafische Darstellung eignet sich hervorragend für eine Auswertung anhand eines Bildauswertealgorithmus.

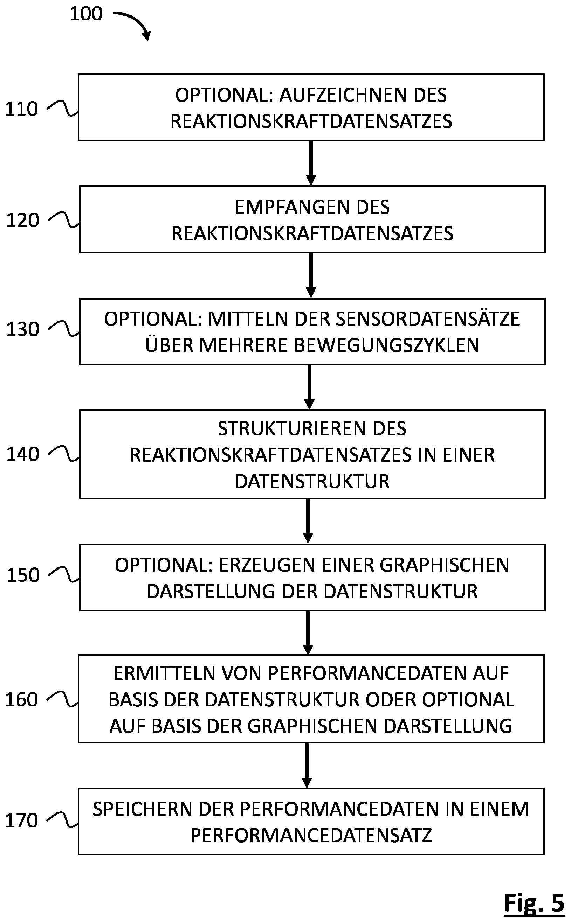

Fig. 5 zeigt ein Ablaufschema eines Ausführungsbeispiels des erfindungsgemäßen Verfahrens 100. In einem optionalen ersten Schritt 110 wird ein Reaktionskraftdatensatz mittels einer auf der Unterseite zumindest eines Fußes des Lebewesens angeordneten Sensoreinrichtung 2 aufgezeichnet, wobei die Sensoreinrichtung 2 ein erstes Paar 2 und ein zweites Paar 6 von bezüglich einer Symmetrieebene E der Sensoreinrichtung 2 zueinander spiegelsymmetrisch angeordneter Sensoren 4a, 4b; 6a, 6b aufweist und der Reaktionskraftdatensatz pro Sensor 4a, 4b; 6a, 6b jeweils einen dem jeweiligen Sensor 4a, 4b; 6a, 6b zugeordneten Sensordatensatz 14a, 14b; 16a, 16b enthaltend einen während der Bewegung aufgezeichneten zeitlichen Verlauf einer mit dem Sensor 4a, 4b; 6a, 6b gemessenen Reaktionskraft aufweist. In einem zweiten Schritt 120 wird der Reaktionskraftdatensatz empfangen. Beispielsweise wird der Reaktionskraftdatensatz von einer Auswerteeinheit empfangen. In einem optionalen dritten Schritt 130 können die Sensordatensätze 14a, 14b; 16a, 16b über mehrere Bewegungszyklen, z.B. Schritte, gemittelt werden. In einem vierten Schritt wird der Reaktionskraftdatensatz in einer Datenstruktur 10 mit zumindest zwei Dimensionen d1, d2 strukturiert (vgl. Fig. 1b). Hierbei werden die Messzeitpunkte der Reaktionskraft entlang einer ersten Dimension d1 und die unterschiedlichen Sensordatensätze 14a, 14b; 16a, 16b entlang einer zweiten Dimension d2 geordnet, derart, dass die dem ersten Paar 4 von Sensoren 4a, 4b zugeordneten Sensordatensätze 14a, 14b bezüglich einer gedachten, in Richtung der ersten Dimension d1 verlaufenden Symmetrieachse A spiegelsymmetrisch gegenüberliegend angeordnet sind und die dem zweiten Paar 6 von Sensoren 6a, 6b) zugeordneten Sensordatensätze 16a, 16b bezüglich der Symmetrieachse A spiegelsymmetrisch gegenüberliegend sowie die dem ersten Paar 4 von Sensoren 4a, 4b zugeordneten Sensordatensätze 14, 14b umgebend angeordnet sind. In einem optionalen fünften Schritt 150 wird auf Basis der Datenstruktur 10 eine graphische Darstellung 20 des Reaktionskraftdatensatzes erzeugt. In einem sechsten Schritt 160 werden Performancedaten für die Bewegung auf Basis der Datenstruktur, gegebenenfalls auf Basis der graphischen Darstellung 20 ermittelt. In einem letzten Schritt 170 werden die Performancedaten in einem Performancedatensatz gespeichert.

Ergänzend sei darauf hingewiesen, dass "aufweisend" keine anderen Elemente oder Schritte ausschließt und "ein" oder "eine" keine Vielzahl ausschließt.

Der Schutzbereich der vorliegenden Erfindung ist durch die Patentansprüche gegeben und wird durch die in der Beschreibung erläuterten oder den Figuren gezeigten Merkmale nicht beschränkt.

## Patentansprüche

1. Computerimplementiertes Verfahren (100) zur Erfassung eines Performancedatensatzes für eine von einem Lebewesen, insbesondere einem Huftier oder einem Menschen, durchgeführte Bewegung enthaltend folgende Schritte:
- Empfangen eines mittels einer auf der Unterseite zumindest eines Fußes des Lebewesens angeordneten Sensoreinrichtung (2) gemessenen Reaktionskraftdatensatzes (Schritt 120),
wobei die Sensoreinrichtung (2) ein erstes und ein zweites Paar (4, 6) von bezüglich einer Symmetrieebene (E) der Sensoreinrichtung (2) zueinander spiegelsymmetrisch angeordneter Sensoren (4a, 4b; 6a, 6b) aufweist,
wobei der Reaktionskraftdatensatz pro Sensor (4a, 4b; 6a, 6b) jeweils einen dem jeweiligen Sensor (4a, 4b; 6a, 6b) zugeordneten Sensordatensatz (14a, 14b; 16a, 16b) enthaltend einen während der Bewegung aufgezeichneten zeitlichen Verlauf einer mit dem Sensor (4a, 4b; 6a, 6b) gemessenen Reaktionskraft aufweist;
- Strukturieren des Reaktionskraftdatensatzes in einer Datenstruktur (10) mit zumindest zwei Dimensionen (d1, d2) (Schritt 140), wobei entlang einer ersten Dimension (d1) die Messzeitpunkte der Reaktionskraft geordnet werden und entlang einer zweiten Dimension (d2) die unterschiedlichen Sensordatensätze (14a, 14b; 16a, 16b) geordnet werden, derart, dass
die dem ersten Paar (4) von Sensoren (4a, 4b) zugeordneten Sensordatensätze (14a, 14b) bezüglich einer gedachten, in Richtung der ersten Dimension (d1) verlaufenden Symmetrieachse (A) spiegelsymmetrisch gegenüberliegend angeordnet sind und
die dem zweiten Paar (6) von Sensoren (6a, 6b) zugeordneten Sensordatensätze (16a, 16b) bezüglich der Symmetrieachse (A) spiegelsymmetrisch gegenüberliegend sowie die dem ersten Paar (4) von Sensoren (4a, 4b) zugeordneten Sensordatensätze (14, 14b) umgebend angeordnet sind;
- Ermitteln von Performancedaten auf Basis der Datenstruktur (10) (Schritt 160) und speichern in einem Performancedatensatz (Schritt 170).

2. Verfahren (100) nach Anspruch 1, wobei die Datenstruktur (10) eine Matrix mit zumindest zwei Indizes ist, wobei Einträge mit dem gleichen ersten Index der Matrix demselben Messzeitpunkt (t) der Reaktionskraft zugeordnet sind und Einträge mit dem gleichen zweiten Index zum gleichen Sensordatensatz (14a, 14b; 16a, 16b) gehören.

3. Verfahren (100) nach Anspruch 1 oder 2, wobei auf Basis der Datenstruktur (10) eine graphische Darstellung (20) des Reaktionskraftdatensatzes erzeugt wird, wobei das Ermitteln von Performancedaten auf Basis der Datenstruktur (10) (Schritt 160) eine Auswertung der graphischen Darstellung (20), insbesondere mittels eines Bildauswertealgorithmus, umfasst.

4. Verfahren (100) nach einem der vorangehenden Ansprüche, wobei die Sensordatensätze (14a, 14b; 16a, 16b) jeweils einen über mehrere von dem Lebewesen durchgeführte Bewegungszyklen gemittelten zeitlichen Verlauf der mit dem jeweiligen Sensor (4a, 4b; 6a, 6b) aufgezeichneten Reaktionskraft enthalten.

5. Verfahren (100) nach einem der vorangehenden Ansprüche, wobei das erste Paar (4) und/oder das zweite Paar (6) von Sensoren auf zwei separate, während der Messung des Reaktionskraftdatensatzes auf der Unterseite unterschiedlicher Füße des Lebewesens angeordneten Sensorträgern (2a, 2b) der Sensoreinrichtung (2) angeordnet ist oder auf einem gemeinsamen, während der Messung des Reaktionskraftdatensatzes auf der Unterseite eines Fußes des Lebewesens angeordneten Sensorträger (2c) der Sensoreinrichtung (2) angeordnet ist oder.

6. Computerprogrammprodukt, umfassend Befehle, die bei der Ausführung des Programms durch einen Computer diesen veranlassen, das Verfahren (100) nach einem der Ansprüche 1 bis 5 auszuführen.

7. Computer, umfassend Mittel zur Ausführung des Verfahrens (100) nach einem der Ansprüche 1 bis 5.

## Claims

1. Computer-implemented method (100) for acquiring a performance data set for a movement performed by a living being, in particular a hoofed animal or a human, comprising the following steps:
- receiving a reaction force data set measured by means of a sensor device (2) arranged on the underside of at least one foot of the living being (step 120),
wherein the sensor device (2) comprises a first and a second pair (4, 6) of sensors (4a, 4b; 6a, 6b) arranged mirror-symmetrically with respect to a plane of symmetry (E) of the sensor device (2),
wherein the reaction force data set, per sensor (4a, 4b; 6a, 6b), in each case comprises a sensor data set (14a, 14b; 16a, 16b) assigned to the respective sensor (4a, 4b; 6a, 6b), said sensor data set comprising a temporal profile, recorded during the movement, of a reaction force measured by means of the sensor (4a, 4b; 6a, 6b);
- structuring the reaction force data set in a data structure (10) having at least two dimensions (d1, d2) (step 140), wherein the measurement time points of the reaction force are ordered along a first dimension (d1) and the different sensor data sets (14a, 14b; 16a, 16b) are ordered along a second dimension (d2), such that
the sensor data sets (14a, 14b) assigned to the first pair (4) of sensors (4a, 4b) are arranged opposite one another in a mirror-symmetrical manner with respect to an imaginary axis of symmetry (A) extending in the direction of the first dimension (d1), and
the sensor data sets (16a, 16b) assigned to the second pair (6) of sensors (6a, 6b) are arranged opposite one another in a mirror-symmetrical manner with respect to the axis of symmetry (A) and surrounding the sensor data sets (14, 14b) assigned to the first pair (4) of sensors (4a, 4b);
- determining performance data on the basis of the data structure (10) (step 160) and storing said performance data in a performance data set (step 170).

2. Method (100) according to claim 1, wherein the data structure (10) is a matrix having at least two indices, wherein entries having the same first index of the matrix are assigned to the same measurement time point (t) of the reaction force and entries having the same second index belong to the same sensor data set (14a, 14b; 16a, 16b).

3. Method (100) according to claim 1 or 2, wherein a graphical representation (20) of the reaction force data set is generated on the basis of the data structure (10), wherein determining performance data on the basis of the data structure (10) (step 160) comprises evaluating the graphical representation (20), in particular by means of an image evaluation algorithm.

4. Method (100) according to one of the preceding claims, wherein the sensor data sets (14a, 14b; 16a, 16b) each contain a temporal profile, averaged over a plurality of movement cycles performed by the living being, of the reaction force recorded by means of the respective sensor (4a, 4b; 6a, 6b).

5. Method (100) according to one of the preceding claims, wherein the first pair (4) and/or the second pair (6) of sensors is arranged on two separate sensor carriers (2a, 2b) of the sensor device (2), which sensor carriers are arranged, during measurement of the reaction force data set, on the underside of different feet of the living being, or is arranged on a common sensor carrier (2c) of the sensor device (2), which sensor carrier is arranged, during measurement of the reaction force data set, on the underside of one foot of the living being, or.

6. Computer program product comprising instructions which, when the program is executed by a computer, cause the computer to carry out the method (100) according to one of claims 1 to 5.

7. Computer comprising means for carrying out the method (100) according to one of claims 1 to 5.

## Revendications

1. Procédé informatisé (100) de saisie d'un jeu de données de performance pour un mouvement exécuté par un être vivant, en particulier un animal ongulé ou un être humain, comprenant les étapes suivantes :
- réception d'un jeu de données de force de réaction mesuré au moyen d'un dispositif de capteurs (2) disposé sur la face inférieure d'au moins un pied de l'être vivant (étape 120),
le dispositif de capteurs (2) comprenant une première et une seconde paire (4, 6) de capteurs (4a, 4b ; 6a, 6b) disposés de manière symétrique en miroir l'un par rapport à l'autre par rapport à un plan de symétrie (E) du dispositif de capteurs (2),
le jeu de données de force de réaction comprenant, pour chaque capteur (4a, 4b ; 6a, 6b), respectivement un jeu de données de capteur (14a, 14b ; 16a, 16b) associé au capteur respectif (4a, 4b ; 6a, 6b), ledit jeu de données de capteur comprenant une évolution temporelle, enregistrée pendant le mouvement, d'une force de réaction mesurée au moyen du capteur (4a, 4b ; 6a, 6b) ;
- structuration du jeu de données de force de réaction dans une structure de données (10) ayant au moins deux dimensions (d1, d2) (étape 140), les instants de mesure de la force de réaction étant ordonnés le long d'une première dimension (d1) et les différents jeux de données de capteurs (14a, 14b ; 16a, 16b) étant ordonnés le long d'une seconde dimension (d2), de telle sorte que les jeux de données de capteurs (14a, 14b) associés à la première paire (4) de capteurs (4a, 4b) sont disposés en vis-à-vis de manière symétrique en miroir par rapport à un axe de symétrie imaginaire (A) s'étendant dans la direction de la première dimension (d1), et
les jeux de données de capteurs (16a, 16b) associés à la seconde paire (6) de capteurs (6a, 6b) sont disposés en vis-à-vis de manière symétrique en miroir par rapport à l'axe de symétrie (A) et en entourant les jeux de données de capteurs (14, 14b) associés à la première paire (4) de capteurs (4a, 4b) ;
- détermination de données de performance sur la base de la structure de données (10) (étape 160) et stockage dans un jeu de données de performance (étape 170).

2. Procédé (100) selon la revendication 1, dans lequel la structure de données (10) est une matrice ayant au moins deux indices, des entrées ayant le même premier indice de la matrice étant associées au même instant de mesure (t) de la force de réaction et des entrées ayant le même second indice appartenant au même jeu de données de capteur (14a, 14b ; 16a, 16b).

3. Procédé (100) selon la revendication 1 ou 2, dans lequel une représentation graphique (20) du jeu de données de force de réaction est générée sur la base de la structure de données (10), la détermination de données de performance sur la base de la structure de données (10) (étape 160) comprenant une évaluation de la représentation graphique (20), en particulier au moyen d'un algorithme d'évaluation d'image.

4. Procédé (100) selon l'une des revendications précédentes, dans lequel les jeux de données de capteurs (14a, 14b ; 16a, 16b) contiennent chacun une évolution temporelle, moyennée sur plusieurs cycles de mouvement exécutés par l'être vivant, de la force de réaction enregistrée au moyen du capteur respectif (4a, 4b ; 6a, 6b).

5. Procédé (100) selon l'une des revendications précédentes, dans lequel la première paire (4) et/ou la seconde paire (6) de capteurs est disposée sur deux supports de capteurs séparés (2a, 2b) du dispositif de capteurs (2), disposés pendant la mesure du jeu de données de force de réaction sur la face inférieure de pieds différents de l'être vivant, ou est disposée sur un support de capteurs commun (2c) du dispositif de capteurs (2), disposé pendant la mesure du jeu de données de force de réaction sur la face inférieure d'un pied de l'être vivant, ou.

6. Produit programme d'ordinateur comprenant des instructions qui, lorsque le programme est exécuté par un ordinateur, amènent celui-ci à exécuter le procédé (100) selon l'une des revendications 1 à 5.

7. Ordinateur comprenant des moyens pour exécuter le procédé (100) selon l'une des revendications 1 à 5.
